# EUROPEAN PATENT APPLICATION

(11) **EP 2 873 375 A1**
(43) Date of publication of application: **20.05.2015**
(21) Application number: 14191782.3
(22) Date of filing: 05.11.2014
(51) Int. Cl.: A61B 17/00, A61M 35/00, B05C 17/00

(54) **Cap-dispenser assembly**

(30) Priority: 13.11.2013 TW 102141163
(71) Applicant: GeneJet Biotech Co., Ltd., Taipei City 11494 (TW)
(72) Inventor: Chu, Fa-Ter, 105 Taipei City (TW)
(74) Representative: Lang, Christian

(57) **Abstract**

A cap-dispenser assembly (3) including a cap (1) and a dispenser (2) attached to the cap (1). The cap (1) is a hollow cylinder with a first pipe (12) provided on the upper surface (104) of the base (10) of the cap (1). A plurality of ribs (16) are provided between the first pipe (12) and the base (10) of the cap (1). The space inside the first pipe (12) and the space inside the base (10) form a fluid channel (18). The dispenser (2) has an upper portion (22) and a lower portion (25). The upper portion (22) of the dispenser (2) is upward tapered while the lower portion (25) of the dispenser (2) is provided at bottom with a recess (26) to receive the first pipe (12) of the cap (1). The cap-dispenser assembly (3) can be attached to a flexible tube to enable dispense of the content in the flexible tube on wounds of various sizes via the fluid channel (18) and pores of the dispenser (2).

## Description

### CROSS-REFERENCE TO RELATED APPLICATIONS

The present invention claims benefit and priority of Taiwanese Patent Application No. TW 102141163, filed on November 13, 2013, which is herein incorporated by reference in its entirety.

### Technical Field of the Invention

The present invention relates to a cap-dispenser assembly, and more particularly to an assembly configured to be used on flexible tubes and assist in dispensing content in the flexible tubes.

### Background of the Invention

It is a common surgical treatment to stitch wounds with suture. Such technique has been developed for a long time. However, no matter how much the suture material has been changed or the stitching technique has been improved, patients still need to endure the pain during stitching and face possible scars after the stitching. In addition, in a case that anesthetics cannot be used or not suitable in treating wounds with plenty nerves or wounds on the face, the aforementioned issues can be much troublesome. Recently, medical adhesives are often used to address the aforementioned issues.

Instant adhesives used as medical adhesives are typically cyanoacrylate compounds. Such cyanoacrylate compounds have characteristic of non toxic, strong adhesion, and not interfering the healing of wounds.

Medical adhesives typically have high polarities, and tissues of organisms are also substances with high polarities, therefore high strength will be created when they are bonded together. Alkyl α-cyanoacrylate is well known among medical adhesives, and its characteristics are extensively studied as well. Usually, Alkyl α-cyanoacrylate can slough off from the skin in about a week.

Typically, the polymerization rate of α-cyanoacrylate decreases as the carbon chain length of the ester group increases. However, as the carbon chain length of ester group increases, the tensile strength of α-cyanoacrylate decreases while the impact resistance of the materials increases.

Currently, the medical adhesives in use include Alkyl-2-cyanoacrylate, alkenyl-2-cyanoacrylate, alkoxyalkyl-2-cyanoacrylate, carbalkoxyalkyl-2-cyanoacrylate, Methyl-2-cyanoacrylate, ethyl-2-cyanoacrylate, n-propyl-2-cyanoacrylate, iso-propyl-2-cyanoacrylate, n-butyl-2-cyanoacrylate, iso-butyl-2-cyanoacrylate, hexyl-2-cyanoacrylate, n-octyl-2-cyanoacrylate, 2-octyl-2-cyanoacrylate, 2-methoxyethyl-2-cyanoacrylate, 2-ethoxyethyl-2-cyanoacrylate, 2-propoxyethyl-2-cyanoacrylate, and a mixture thereof.

In addition, some thickeners may be added to the aforementioned compounds. In order not to influence the properties of the compounds, polymers being compatible with cyanoacrylates may be used as thickeners, such as n-amylcyanoacrylate, isoamylcyanoacrylate, decylcyanoacrylate, 3-acetoxypropylcyanoacrylate, 2-methoxypropylcyanoacrylate, 3-chloropropylcyanoacrylate, benzylcyanoacrylate, phenylcyanoacrylate, fluorinated 2-cyanoacrylates.

Further, some plasticizer may also be added to the medical adhesive, such as tributylcitrate, acetyltributylcitrate, dimethylsebacate, diethylsebacate, triethylphosphate, tri(2-ethyl-hexyl)phosphate, tri(p-cresyl)phosphate, glyceryltriacetate, glyceryltributyrate, dioctyladipate, isopropylmyristate, butylstearate, lauricacid, trioctyltrimellitate, dioctylglutarate.

After being applied to a wound or incision on a human body, the medical adhesive forms a protective film with high binding strength in a few seconds. Such binding strength enables the wound to bear a tension force of more than 50 Newtons per square centimeter. The protective film will slough off in about a week. Medical adhesives can protect wounds, inhibit bacteria and accelerate wound healing, without causing any allergic reaction to the skin. Furthermore, patients having their wounds treated with medical adhesives have better living quality than those treated by conventional surgical stitching. In addition to superficial wounds, medical adhesives can also be used to facilitate the healing of stitched wounds, totreat wound for athletes, or to bind bones and cartilages. As a result, medical adhesives are important tools for surgeons.

Similar to ointments, the medical adhesive is viscous liquid and typically stored in a container before being squeezed out for use. The container for the medical adhesive is usually a flexible tube. By squeezing the flexible tube, the content therein can be conveniently squeezed out and dispensed on a targeted object.

The issued US patent No. 8,123,426 disclosed a dispenser to be used on flexible tubes, wherein a cap for dispensing can be separated into three components. Apparently, the structure of the dispenser is complicated and difficult to be manufactured. In addition, the design of the dispenser does not facilitate dispensing the content on a small wound.

Taiwanese published patent publication No. 201306889 disclosed a dispensing container which is also composed of three components, and is therefore difficult to manufacture. Although the dispenser having an elongated shape is convenient to use, the strength of the dispenser may be insufficient so that the dispenser could be snapped easily.

In addition to the problems set forth above, in a case of dispensing ointment or medical adhesives, the part of the dispensing device that directly contacts the wound should be replaced after each use. Moreover, the dispensing device loses its function to dispense the adhesive when the adhesive is cured in the device. As a result, the dispensing device needs to be replaced frequently to ensure its normal operation. Apparently, the dispensers as mentioned above are not suitable for being replaced after each use. Therefore, it is necessary to provide an improved dispenser for addressing the abovementioned problems.

### SUMMARY OF THE INVENTION

In view of the problems mentioned above, the invention provides a cap-dispenser assembly having a cap with simple but strengthened structure and an easy-to-be-replaced dispenser. The cap can be used for dispensing content in any flexible tubes in practical use.

An object of the invention is to provide a cap-dispenser assembly including only a cap and a dispenser with the following features. The cap includes at least a base, a first pipe, and a plurality of ribs. The base is a hollow column formed with an open end and a closed end distant from the open end. The closed end is provided with a through hole. An accommodating space is provided within the base. The closed end has an upper surface and a lower surface opposite to the upper surface. The first pipe has a first end surface and a second end surface distant from the first end surface with the second end surface being connected to the upper surface on the closed end of the base. The inside of the first pipe intercommunicates with the accommodating space via the through hole. Each of the ribs has a lower edge and a side edge with the lower edge being connected to the upper surface of the closed end of the base and the side edges being connected to the side surface of the first pipe. The dispenser is a porous column having an upper portion and a lower portion connected sequentially. The upper portion is tapered. The lower portion has a smooth side surface with a recess on the bottom end surface. The cap-dispenser assembly is characterized in that the first end surface of the first pipe is inserted into the recess formed on the bottom end surface of the lower portion of the dispenser and the space inside the first pipe and the through hole forms a fluid channel intercommunicating with the accommodating space. The advantages of the cap-dispenser assembly of the invention are as follows.

The cap-dispenser assembly of the invention enables an user to conveniently dispense medical adhesives to wounds of different sizes via the above-mentioned dispenser.

The cap and the dispenser of the cap-dispenser assembly of the invention comprises two individual components that are easily to be assembled and produced. Additionally, the structure of the cap-dispenser assembly is strengthened to prevent it from breakage in usage.

Moreover, the cap of the cap-dispenser assembly of the invention is designed to allow easy replacement of the dispenser and therefore the cap can be kept clean and workable all the time..

### BRIEF DESCRIPTION OF THE DRAWINGS

FIG. 1A is a perspective view schematically illustrating a cap of a cap-dispenser assembly according to a first embodiment of the invention;
FIG. 1B is a top view schematically illustrating the cap of the cap-dispenser assembly according to the first embodiment of the invention;
FIG. 2A is a perspective view schematically illustrating a dispenser of the cap-dispenser assembly according to the invention;
FIG. 2B is a vertical cross-sectional view schematically illustrating the middle and lower portions of the dispenser of the cap-dispenser assembly according to the invention;
FIG. 3 is a perspective view schematically illustrating the cap-dispenser assembly including the cap and the dispenser according to the first embodiment of the invention;
FIG. 4 is a perspective view schematically illustrating a usage of the cap-dispenser assembly including the cap and the dispenser with a flexible tube according to the first embodiment of the invention; and
FIG. 5 is a perspective view schematically illustrating a cap of a cap-dispenser assembly according to a second embodiment of the invention.

### DETAILED DESCRIPTION OF THE PREFERRED EMBODIMENTS

The objectives, technical features and advantages of the invention would be better understood in the following and enables those skilled in the art to practice the invention. Thus, the technical features and embodiments of the invention are set forth in the following description in conjunction with the accompanying drawings, and preferred embodiments are presented for further illustration. However, the following description with respect to the embodiments is not intended to limit the invention, and the drawings correspondingly referred in the description are intended only to schematically illustrate the features of the invention.

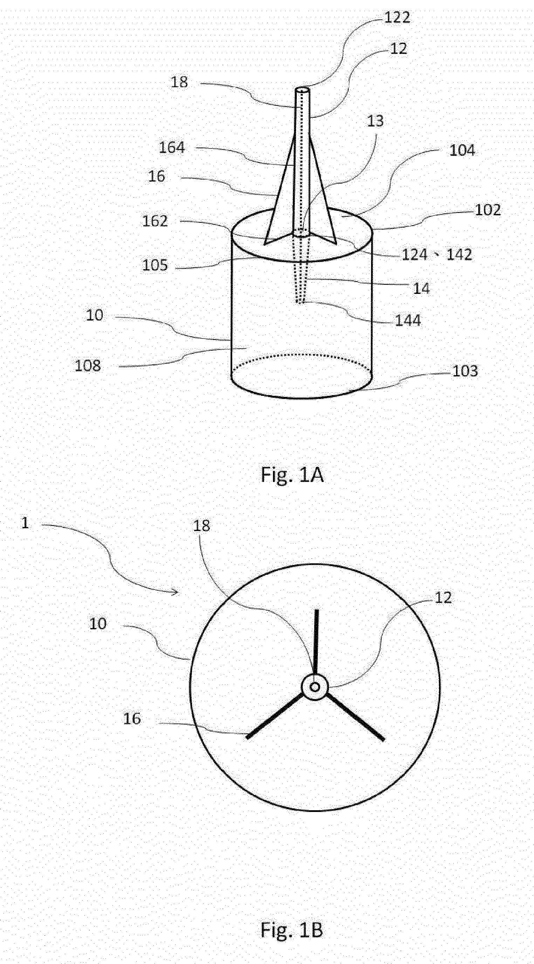

Referring to FIG. 1A, a cap of a cap-dispenser assembly according to a first embodiment of the present invention is illustrated. As shown in FIG. 1A, the cap 1 includes a base 10, a first pipe 12 positioned above and outside the base 10, a second pipe 14 positioned inside the base 10, and a plurality of ribs 16 positioned above the base 10 and around the first pipe 12 to support the first pipe 12. Specifically, the base 10 is a hollow column having an upper end 102 and a lower end 103 distant from the upper end 102. In a preferred embodiment of the invention, the base 10 is a cylinder, but the invention does not limit the geometry shape of the base 10. The base 10 may also be a polygonal column. The upper end 102 of the base 10 is a closed end formed with a through hole 13, and the upper end 102 has an upper surface 104 and a lower surface 105 opposite to the upper surface 104. The lower end 103 of the base 10 is an open end. The hollow portion of the base 10 is formed to be an accommodating space 108 intercommunicating with the lower end 103 of the base 10.

The first pipe 12 has a bottom end surface 124 and a top end surface 122 distant from the bottom end surface 124. The bottom end surface 124 of the first pipe 12 is connected to the upper surface 104 of the upper end 102 of the base 10. The space inside the first pipe 12 intercommunicates with the accommodating space 108 via the through hole 13 formed on the upper end 102 of the base 10. The second pipe 14 is positioned inside the accommodating space 108. The second pipe 14 has a top end surface 142 and a bottom end surface 144 distant from the top end surface 142. The top end surface 142 of the second pipe 14 is connected to the lower surface 105 of the upper end 102 of the base 10. The space inside the second pipe 14 intercommunicates with the space inside the first pipe 12 via the through hole 13 formed on the upper end 102 of the base 10. Therefore, a fluid channel 18 including the space inside the first pipe 12, the through hole 13 formed on the upper end 102 of the base 10, and the space inside the second pipe 14 is formed. The fluid channel 18 is a passage extending from the top end surface 122 of the first pipe 12 to the bottom end surface 144 of the second pipe 14 through the upper end 102 of the base 10, and intercommunicating with the accommodating space 108. In a preferred embodiment, the outer diameter of the second pipe 14 gradually decreases from the top end surface 142 to the bottom end surface 144. In other words, the second pipe 14 tapers toward the bottom end surface 144 of the second pipe 14 and therefore the bottom end of the second pipe 14 is sharpened.

The cap 1 further includes the ribs 16 positioned above the base 10 and around the first pipe 12 to support the first pipe 12. Each of the ribs 16 has a lower edge 162 and a side edge 164 that intersect with each other. The lower edge 162 of each of the ribs 16 is connected to the upper surface 104 of the upper end 102 of the base 10, while the side edge 164 of each of the ribs 16 is connected to the side surface of the first pipe 12. Accordingly, the ribs 16 positioned around the first pipe 12 reinforce the connection between the first pipe 12 and the base 10. Each of the ribs 16 is preferred but not limited to have a triangle shape with the side edge 164 being perpendicular to the lower edge 162. In a preferred embodiment, the cap 1 may have three ribs 16 arranged in an equidistant manner such as shown in FIG. 1B. However, the numbers and arrangement of the ribs 16 of the cap 1 are not limited in the invention.

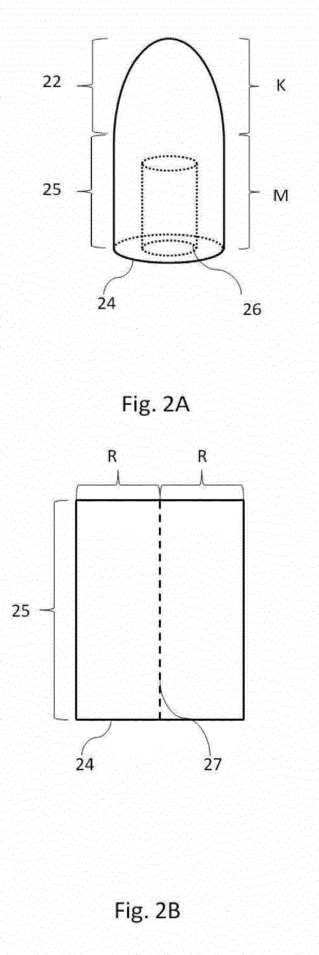

Referring to FIG. 2A, a dispenser of the cap-dispenser assembly according to the invention is illustrated. As shown in FIG. 2A, the dispenser 2 includes an upper portion 22, and a lower portion 25 that are sequentially connected. The lower portion 25 is a column having a smooth side surface with the top of the lower portion 25 being connected to the upper portion 22. In a preferred embodiment, the lower portion 25 is a cylinder. The upper portion 22 is upward tapered to have a narrower top. The bottom end surface 24 of the lower portion 25 is formed to include a recess 26 to properly receive the top end surface 122of the first pipe 12. The length of the upper portion 22 is K, the length of the lower portion 25 is M, and the ratio of K to M ranges from 1:1.5 to 1:4. In a preferred embodiment, the ratio of K to M is 1:2. The bottom end surface 24 of the lower portion 25 can be, but not limited to, a convex, a plane or a concave surface. The dispenser 2 is made of air permeable material that is porous or has a plurality of pores (not shown), such as silica gel, polyurethane (PU).

Referring to FIG. 2B, the lower portion 25 of the dispenser 2 of the cap-dispenser assembly according to the invention is illustrated. As shown in FIG. 2B, a cylinder having a smooth side surface is exemplified as the lower portion 25 of the dispenser 2 , the horizontal distance between any point on the central axis 27 of the lower portion 25 and the side surface of the lower portion 25 will be a constant value R.

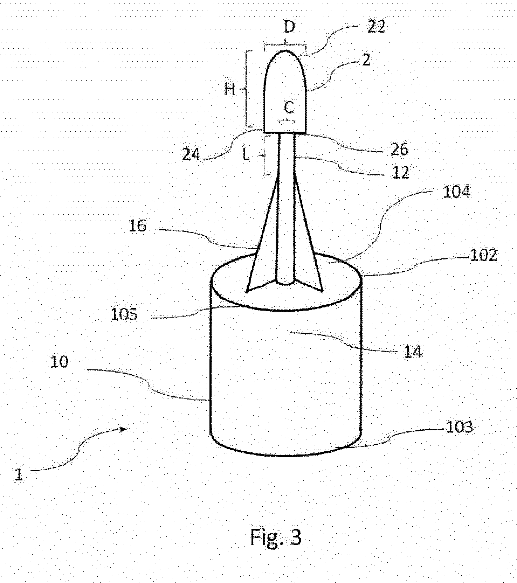

Referring to FIG. 3, the cap-dispenser assembly according to the first embodiment of the invention is illustrated. As shown in FIG. 3, the cap-dispenser assembly 3 according to one embodiment includes the cap 1 as shown in FIG. 1A and the dispenser 2 as shown in FIG. 2A, wherein the top end surface 122 of the first pipe 12 is inserted into and therefore received by the recess 26 formed on the bottom end surface 24 of the lower portion 25 of the dispenser 2 as shown in Fig. 2A. Moreover, the radial cross-sectional outer diameter of the dispenser 2 is D, the length of the dispenser 2 including the upper portion 22 and the lower portion 25 is H, and the outer diameter of the top end surface 122 of the first pipe 12 is C. In a preferred embodiment, the relationship between H, D and C is H>D>C. In addition, in a preferred embodiment, after the dispenser 2 is attached to the first pipe 12, the bottom end surface 24 of the dispenser 2 is distant from the top end of the side edge 164 of each of the ribs 16 with a distance L.

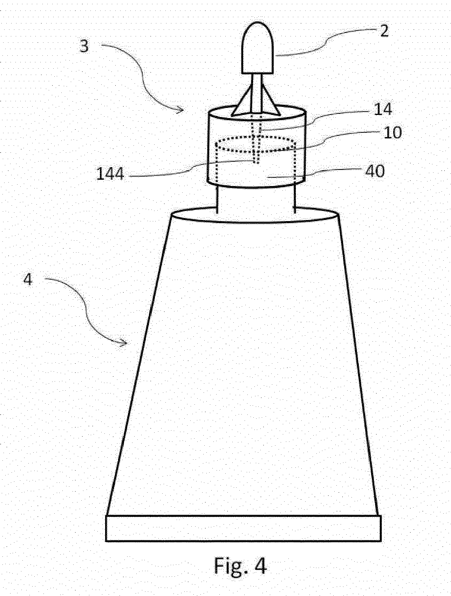

Referring to FIG. 4, a usage of the cap-dispenser assembly with a flexible tube according to the first embodiment of the present invention is illustrated. As shown in FIG. 4, a tube orifice section 40 of a flexible tube 4 is fitly received in the accommodating space 108 of the base 10 such that the cap-dispenser assembly 3 is attached to the flexible tube 4. Specifically, the outer surface of the tube orifice section 40 is formed with male threads (not shown), while the inner surface of the base 10 is formed with female threads (not shown) to be correspondingly engaged with the male threads on the tube orifice section 40. In this way, when the cap-dispenser assembly 3 is attached to the flexible tube 4, the second pipe 14 inside the accommodating space 108 of the base 10 is inserted into the tube orifice section 40. As a result, the content (not shown) in the flexible tube 4 will flow into the dispenser 2 through the fluid channel 18 including the space inside the second pipe 14, the through hole 13 formed on the upper end 102 of the base 10, and the space inside the first pipe 12 (as shown in FIG. 1A and FIG. 1 B) when the flexible tube 4 is squeezed. As mentioned above, since the dispenser 2 is made of air permeable materials having a plurality of pores, the content (not shown) in the flexible tube 4 permeate through the pores of the dispenser 2 and spread onto the surface of the dispenser 2. Therefore, a user can dispense the content (not shown) on the surface of the dispenser 2 to a wound. In addition, as mentioned above, the upper portion 22 of the dispenser 2 is upward tapered, and the total length H of the dispenser 2 (i.e., K+M) is larger than the radial cross-sectional outer diameter D of the dispenser 2, and the radial cross-sectional outer diameter D of the dispenser 2 is larger than the outer diameter C of the first pipe 12 at the top end surface 122. Thus, when the cap-dispenser assembly 3 is used to dispense content on a larger wound, the smooth side surface of the lower portion 25 of the dispenser 2 can be used to facilitate the treatment of the large wound. On the other hand, when the cap-dispenser assembly 3 is used in dispensing a wound of a small size, the tapered upper portion 22 of the dispenser 2 can be used to facilitate a precise treatment of the small wound.

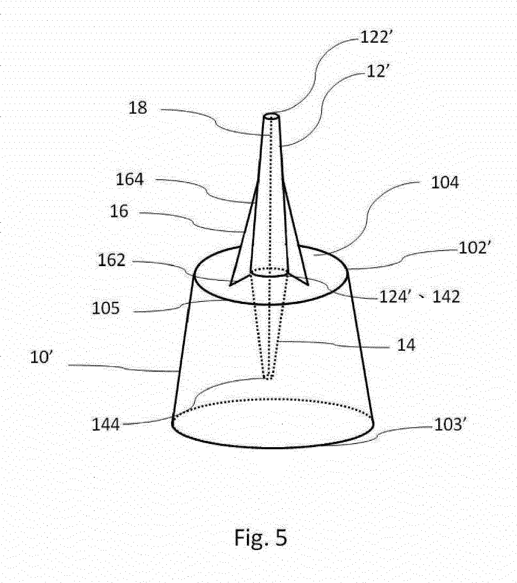

Referring to FIG. 5, a cap 1' of a cap-dispenser assembly according to a second embodiment of the invention is illustrated. As shown in FIG. 5, the base 10' of the cap 1' is similar to the base 10 of the cap 1 except that the upper end 102' of the base 10' of the cap 1' has an outer diameter smaller than that of the lower end 103'. Such design for the base 10' enables the base 10' to be easily held by a user. Moreover, the outer diameter of the top end surface 122' of the first pipe 12' is smaller than that of the bottom end surface 124' of the first pipe 12', i.e., the first pipe 12' is upward tapered. This structure reinforces the connection between the first pipe 12' and the base 10'. Other features of the cap 1' are the same as that of the cap 1 as shown in FIG. 1A, and thus will not be described redundantly.

With the cap-dispenser assembly including the cap 1 or 1' and dispenser 2 according to the invention, the user is able to dispense viscous content such as medical adhesive in the flexible tube 4 with ease. In addition, the dispenser 2 of the cap-dispenser assembly can be conveniently replaced, and therefore the cap of the cap-dispenser assembly can function all the time without being affected by a clotted dispenser 2. Furthermore, the dispenser 2 of the invention is designed to allow a user to dispense medical adhesives to wounds of different sizes. Moreover, the ribs 16 reinforce the structural strength of the cap 1 or cap 1' such that the first pipe 12 or first pipe 12' is firmly supported and would not be easily broken or snapped in usage. Additionally, the dispose of the ribs 16 increases the friction and enables the user to easily apply force on the cap 1 or the cap 1' and therefore insert the bottom end surface 144 of the second pipe 14 into the tube orifice section 40 of the flexible tube 4 when the user rotate the cap 1 or the cap 1' onto the flexible tube 4.

The cap 1 or the cap 1' and the dispenser 2 of the cap-dispenser assembly of the invention has two components that are easy to be assembled. Thus, the cap-dispenser assembly 3 of the invention can be readily mass produced, and can be widely applied to any flexible tubes of standard sizes.

## Claims

1. A cap-dispenser assembly (3) comprising:
a cap (1) including:
a base (10) being a hollow column formed with an open end (103) and a closed end (102) distant from the open end (103), the closed end (102) being provided with a through hole (13), an accommodating space (108) being formed within the base (10), the closed end (102) having an upper surface (104) and a lower surface (105) opposite to the upper surface (104);
a first pipe (12) having a first end surface (122) and a second end surface (124) distant from the first end surface (122) with the first end surface (122) being open and the second end surface (124) being connected to the upper surface (104) of the closed end (102) of the base (10), the inside of the first pipe (12) intercommunicating with the accommodating space (108) via the through hole (13); and
a plurality of ribs (16) each having a lower edge (162) and a side edge (164) with the lower edges (162) being connected to the upper surface (104) of the closed end (102) of the base (10) and the side edges (164) being connected to a side surface of the first pipe (12); and
a dispenser (2) including a plurality of pores, having an upper portion (22) and a lower portion (25), the lower portion (25) being a column with a smooth side surface, the top of the lower portion (25) being connected to the upper portion (22), the upper portion (22) being upward tapered, a bottom end surface (24) of the lower portion (25) being formed with a recess (26);
wherein the first end surface (122) of the first pipe (12) is inserted into the recess (26) formed on the bottom end surface (24) of the lower portion (25) of the dispenser (2); and
wherein the space inside the first pipe (12) and the through hole (13) forms a fluid channel (18) intercommunicating with the accommodating space (108).

2. The cap-dispenser assembly (3) of claim 1, wherein the cap (1) further includes a second pipe (14) inside the accommodating space (108), the second pipe (14) has a first end surface (142) and a second end surface (144) distant from the first end surface (142) with the first end surface (142) being connected to the lower surface (105) of the closed end (102) of the base (10), the space inside the second pipe (14) intercommunicates with the space inside the first pipe (12) via the through hole (13), and the fluid channel (18) includes the space inside the second pipe (14).

3. The cap-dispenser assembly (3) of claim 2, wherein the outer diameter of the second pipe (14) gradually decreases from the first end surface (142) to the second end surface (144).

4. The cap-dispenser assembly (3) of claim 1, wherein the dispenser (2) is made of an air permeable material selected from the group consisting of silica gel and polyurethane.

5. The cap-dispenser assembly (3) of claim 1, wherein the length of the dispenser (2) including the upper portion (22) and the lower portion (25) is larger than a radial cross-sectional outer diameter of the dispenser (2).

6. The cap-dispenser assembly (3) of claim 5, wherein the radial cross-sectional outer diameter of the dispenser (2) is larger than the outer diameter of the first pipe (12) at the first end surface (122).

7. The cap-dispenser assembly (3) of claim 1, wherein the bottom end surface (24) of the lower portion (25) of the dispenser (2) is distant from the top end of the side edge (164) of each of the ribs (16).

8. The cap-dispenser assembly (3) of claim 1, wherein the outer diameter of the first pipe (12) at the first end surface (122) is smaller than the outer diameter of the first pipe (12) at the second end surface (124).

9. The cap-dispenser assembly (3) of claim 1, wherein the outer diameter of the closed end (102) of the base (10) is smaller than the outer diameter of the open end (103) of the base (10).
